# EUROPEAN PATENT APPLICATION

(11) **EP 1 270 012 A1**
(43) Date of publication of application: **02.01.2003**
(21) Application number: 02253950.6
(22) Date of filing: 06.06.2002
(51) Int. Cl.: A61K 38/28, A61K 9/00, A61P 3/10, A61P 5/50

(54) **Use of pulmonary administration of insulin for treatment of diabetes**

(30) Priority: 21.06.2001 US 300049 P
(71) Applicant: Pfizer Products Inc., Groton, Connecticut 06340 (US)
(72) Inventor: Landschulz, William Harras, New London, Connecticut 06320 (US)
(74) Representative: Wood, David John

(57) **Abstract**

Method of decreasing fasting sugars and weight gains in diabetic patients.

A method of reducing the rate of weight gain, or lowering the fasting level, in a diabetic patient who requires exogenous insulin, comprising administering insulin to said patient by the pulmonary route.

## Description

### Field of the Invention

This invention is directed to a method of reducing the rate of weight gain and/or to lowering fasting blood sugars in a diabetic patient who is using exogenous insulin to control blood sugars, and who is taking said insulin by other than a pulmonary route of administration, comprising administering said insulin to said patient by the pulmonary route, i.e. as inhaled insulin. Additionally, the invention relates to starting a patient on inhaled insulin who is at risk for gaining weight or developing high fasting blood sugars.

### Background of the Invention

Diabetes mellitus is a serious metabolic disease that is defined by the presence of chronically elevated levels of blood glucose. Classic symptoms of diabetes mellitus in adults are polyuria and polydipsia together with elevated levels of plasma glucose. Normal fasting plasma glucose concentrations are less than 110 milligrams per deciliter. In diabetic patients, fasting concentrations are found to be at or above 126 milligrams per deciliter. In general, diabetes mellitus develops in response to damage to, or to defects in, the beta cells of the pancreas.

Primary diabetes mellitus is classified as Type 1 diabetes (also called insulin-dependent diabetes mellitus or IDDM) and Type 2 diabetes mellitus (also called non-insulin dependent diabetes mellitus or NIDDM). Type I (juvenile onset or insulin-dependent) diabetes is a well-known hormone deficient state, in which the pancreatic beta cells appear to have been destroyed by the body's own immune defense mechanisms. Patients with Type I diabetes mellitus have little or no endogenous insulin secretory capacity. These patients develop extreme hyperglycemia. Type I diabetes was fatal until the introduction of insulin replacement therapy some 70 years ago - first using insulins from animal sources, and more recently, using human insulin made by recombinant DNA technology.

Type 2 diabetes is characterized by insulin resistance, i.e., a failure of the normal metabolic response of peripheral tissues to the action of insulin, compounded by a relative insulin deficiency. In other words, insulin resistance is a condition where the circulating insulin produces a subnormal biological response. In clinical terms, insulin resistance is present when normal or elevated blood glucose levels persist in the face of normal or elevated levels of insulin. The hyperglycemia associated with Type 2 diabetes can sometimes be reversed or ameliorated by diet or weight loss sufficient to restore the sensitivity of the peripheral tissues to insulin. Progression of Type 2 diabetes mellitus is associated with increasing concentrations of blood glucose and coupled with a relative decrease in the rate of glucose-induced insulin secretion. Thus, for example, in late-stage Type 2 diabetes mellitus, there may be an insulin deficiency.

The timewise progressions of Type I versus Type 2 diabetes can (and usually do) differ markedly. Youthful (e.g., pediatric) patients suffering from Type I diabetes may not have their condition diagnosed until after the bulk of pancreatic beta cells have been destroyed, thereby necessitating chronic insulin therapy. Usually, Type I progresses on the order of a couple of years before the pancreas has been damaged to the point that it no longer produces sufficient insulin to meet the patient's metabolic needs. In contrast to Type I diabetes, treatment of Type 2 diabetes frequently does not require the use of insulin, and the condition itself can progress over several decades. Institution of therapy in Type 2 diabetes usually involves a trial of dietary therapy and lifestyle modification, typically for 6-12 weeks in the first instance. Features of a diabetic diet include an adequate but not excessive total calorie intake, with regular meals, restriction of the content of saturated fat, a concomitant increase in the polyunsaturated fatty acid content, and an increased intake of dietary fiber. Lifestyle modifications include the maintenance of regular exercise, as an aid both to weight control and also to reduce the degree of insulin resistance. As mentioned above, in late-stage Type 2 diabetes mellitus, there may be a frank insulin deficiency such that, ultimately, a Type 2 patient will require the administration of exogenous insulin to aid in controlling his or her sugar metabolism.

Whether classified as Type 1 or Type 2, diabetic patients suffer special medical problems not characteristic of most non-diabetics. Among the most severe are (1) weight gains which can lead to obesity and further insulin resistance and (2) chronically high levels of fasting glucose. This is particularly true of diabetic patients who self administer insulin by the subcutaneous route, the most common route of administration used by diabetics, although these conditions could also be developed by diabetics who take insulin by a transdermal route. Patients who gain weight run the risk of ultimately becoming obese and suffering the special set of problems associated with becoming obese, namely the general strain on the heart and vasculature together with the increased risk of heart attacks and other heart conditions that accompany obesity and/or significant weight gain. In addition, it is well known that the lower a diabetic patient's fasting glucose is within a normal range, the less likely the patient is to suffer diabetic complications such as retinopathy, neuropathy, stroke, heart attack, kidney disease and/or failure, impotence, and amputation (i.e., of extremities due to poor blood flow and loss of sensation).

### Summary of the Invention

This invention provides a method of reducing the rate of weight gain in a diabetic patient who is using exogenous insulin to control blood sugars and who is (or has been) taking said insulin by a subcutaneous and/or transdermal route of administration, comprising administering said insulin to said patient by the pulmonary route, i.e., as inhaled insulin. Insulin delivered by a pulmonary route is sometimes referred to herein (including the claims) as "pulmonary insulin", which is a synonym for "inhaled insulin".

The invention additionally provides a method of lowering the fasting glucose level in a diabetic patient who is using exogenous insulin to control blood sugars and who is taking said insulin by a subcutaneous and/or transdermal route of administration, comprising administering said insulin to said patient by the pulmonary route.

The invention thus provides a method for reducing the rate of weight gain or for reducing fasting glucose in a diabetic patient who has been taking exogenous insulin either subcutaneously and/or transdermally. In a principle embodiment, the patient is simply switched to a new regimen which requires, in whole or in part, the patient to take his or her insulin via the pulmonary route. The invention is thus considered to be especially applicable to (1) switching diabetic patients, who are already on a regimen of either subcutaneous and/or transdermal insulin, and who are actually suffering from the aforementioned conditions, wholly or partially over to pulmonary insulin; or to (2) starting a newly diabetic patient considered at risk for these conditions out on a regimen which, in whole or in part, requires pulmonary insulin, without ever trying subcutaneous and/or transdermal administration alone, i.e. without pulmonary insulin. It is particularly preferred to practice the invention by switching patients suffering from either or both conditions from a regimen of subcutaneously administered insulin over to a regimen which requires, in whole or in part, that the insulin be administered as inhaled insulin.

The above-mentioned conditions of weight gain and fasting glucose are separate conditions which, in general, must be separately remediated or treated. A medicine or treatment which improves (i.e., lowers) the rate of weight gain, for example, will not necessarily lead to a lowered fasting glucose, and vice-versa. Indeed, improved or even the best glycemic control often leads to increased weight gain in diabetic patients. For this reason, the invention is very surprising in that it provides a method not only for lowering the rate of weight gain, but also for lowering fasting glucose.

A "patient who is taking insulin" includes patients who have been newly diagnosed as diabetic and are about to start on a regimen of insulin to aid in controlling blood sugars. The invention is accordingly applicable to Type 2 as well as Type 1 diabetics.

The phrase "comprising administering said insulin to said patient by the pulmonary route" as it is used above and in the claims will, in many cases apply to switching a patient from the subcutaneous route of insulin administration over to the inhaled/pulmonary route of administration. As disclosed above, the claims also embrace the situation where a patient who is taking exogenous insulin either subcutaneously or transdermally is only partially switched over to inhaled insulin as part of his or her new regimen, and who accordingly still administers insulin subcutaneously and/or transdermally as part of the new regimen. Thus a patient who is administering insulin solely by subcutaneous injection, for example, may, within the scope of the invention, be switched to a regimen which entails taking insulin by for instance, a single injection of subcutaneous long-acting insulin plus pre-meal inhaled insulin.

"Insulin" means the art-recognized polypeptide used in the treatment of diabetics in a substantially purified form, and also the various commercially available forms which include excipients. The term encompasses natural extracted human insulin, recombinantly produced human insulin, insulin extracted from bovine and/or porcine sources, recombinantly produced porcine and bovine insulin, and mixtures thereof. The term "insulin" is also intended to encompass insulin analogs wherein one or more of the amino acids within the polypeptide chain has been replaced with an alternative amino acid and/or wherein one or more of the amino acids has been deleted or wherein one or more additional amino acids has been added to the polypeptide chain. In general, such insulin analogs of the present invention include "super insulin analogs" wherein the ability of the insulin analog to affect serum glucose levels is substantially enhanced as compared with conventional insulin as well as hepatoselective insulin analogs which are more active in the liver than in adipose tissue. The invention may employ an aerosolized inhaled insulin which is monomeric, such as insulin lispro.

"Fasting glucose" means the value of measured glucose in the blood under a particular set of prescribed conditions, for example the level of blood glucose measured after a patient has not eaten, typically in the morning after sleep and before breakfast. It can be measured conventionally by many methods which are well known in the art, many of which are commercially available, for example in the form of kits. A "normal" value for measured fasting glucose is in the range of 80 mg/dl to 126 mg/dl. The lower the value of fasting glucose within this range, the better for the patient. This invention thus provides, inter alia, a method of lowering fasting glucose within this normal range, as well as lowering fasting glucose to within the range when a patient's fasting glucose initially starts out at a valve above 126 mg/dl.

Administering insulin by the "pulmonary route" or as "pulmonary insulin" means administering insulin as inhaled insulin.. "Inhaled insulin", in turn, means an aerosolized, wet or dry, particulate or droplet, insulin-containing formulation which is administered by having the patient "breathe" an insulin-containing aerosol into the lungs, generally by drawing the aerosol through the mouth and into the deep lung. The formulation can, for example, comprise dry particles inhaled from a dry powdered inhaler such as that available from Inhale Therapeutics Systems, San Carlos, CA. The inhaled insulin formulation can also be a particulate, insulin-containing formulation suspended in a propellant. Alternatively, the formulation can be a wet aerosol, i.e., a liquid aerosol of the type produced from an aqueous solution of insulin by a liquid nebulizer system (see Laube, Journal of Aerosol Medicine, Vol 4, No. 3, 1991, and US Patent 5,320,094, herein incorporated by reference in their entirety). The exact aerosol formulation is not believed to be particularly critical, such that the powder can be in the form of a dry powder or a wet insulin-containing aerosol of the type produced by a nebulizer, so long as the particle size, whether liquid or dry, is of a size which facilitates penetration to the deep lung, in which it is believed that the alveoli serve as the portals from the lungs to the blood. Generally, such a particle size is less than about 10 µm. "Inhaled insulin" is to be contrasted with "intranasally administered insulin" in which insulin is administered within the nasal passages and is weakly absorbed into the bloodstream through the nasal mucosa.

Inhaled insulin, in this invention, is employed to reduce the rate of weight gain and the levels of fasting glucose in diabetic patients, meaning that inhaled insulin can be administered to patients who are at risk for either (or both) condition(s). "At risk", can refer to patients who have suffered weight gains and/or high fasting glucose levels in the past and who, therefore, are considered to have risk factors due simply to their medical history of having already suffered from one or both conditions. "At risk" also refers to patients who may not have suffered from a high rate of weight gain or high fasting glucose, but who are otherwise considered to be at risk for these conditions due to other factors such as poor glycemic control, i.e., high glucose levels above the normal range or excess weight gains considered above normal. Such patients may be deemed desirable for initiating on a regimen of inhaled insulin from the start, i.e., without ever commencing administration of insulin subcutaneously or transdermally. If a patient is initiated on inhaled insulin, the patient will generally experience a lower rate of weight gain and/or an ultimate lower absolute body weight once the patient's weight gain has leveled off. The patient will additionally and/or alternatively experience lower average levels of fasting glucose relative to patients self-administering insulin solely by subcutaneous and/or transdermal administration. If a patient is switched over to inhaled insulin as a therapy from self administering insulin subcutaneously, for example, the patient will generally experience a reduction in the rate of weight gain, a stoppage in weight gain, or, in some case, even a reduction in weight, and the phrase "reducing the rate of weight gain" is intended to be inclusive of all such situations. Thus inhaled insulin can either reduce the level of fasting glucose in diabetic patients, reduce the rate of weight gain in such patients, or both, relative to patients on subcutaneously and/or transdermally administered insulin.

It is preferred to administer inhaled insulin from the time that a patient is either diagnosed to be at risk for weight gain and/or high fasting glucose, or actually diagnosed to actually be gaining weight or to have high fasting glucose levels, certainly where the fasting glucose level exceeds 126 mg/dl. Inhaled insulin treatment should be maintained until weight and/or fasting glucose have returned to acceptable levels as determined by the attending physician or, failing such a return to levels deemed satisfactory by the physician, permanently.

### Detailed Description

### A. Inhalers/Administration

Any inhaler known to the art may be used for this invention so long as it is capable of delivering a therapeutically effective dose of insulin to the deep lung. This includes any device such as those which are classified as dry powder inhalers, nebulizers, and metered dose inhalers. Potentially useful are art-recognized inhalers such as those sold under the names Turbohaler (Astra), Rotahaler® (Glaxo), Diskus® (Glaxo), the Ultravent nebulizer (Mallinckrodt), the Acorn II nebulizer (Marquest medical Products), the Ventolin® metered dose inhaler (Glaxo), the Spinhaler® powder inhaler (Fisons), or the like.

In a preferred embodiment, insulin is inhaled as a dry powder by means of a hand-held device such as that disclosed in any of US patents 6,089,228, 5,458,135, 5,775,320, 5,785,049, 5,740,794, and WO 93/00951, the full disclosures of which are herein incorporated by reference. Such devices are available from Inhale Therapeutics Systems, San Carlos, CA.

### B. Formulations

Any formulation which makes it possible to produce aerosolized forms of insulin which can be inhaled and delivered to a patient via the intrapulmonary route can be used in connection with the present invention. Specific information regarding formulations which can be used in connection with aerosolized delivery devices are described within Remington's Pharmaceutical Sciences, A. R. Gennaro editor (latest edition) Mack Publishing Company. Regarding insulin formulations, it is also useful to note Sciarra et al. [*Journal of Pharmaceutical Sciences,* Vol. 65, No. 4, 1976].

A variety of different insulin-containing aerosol formulations can be used in connection with the present invention. The active ingredient within such formulations is insulin which is preferably recombinantly produced human insulin, but which may include insulin extracted from animal sources. Further, the insulin may be an insulin analog which is an analog of human insulin which has been recombinantly produced. Although the insulin and/or analog may be present by itself as the sole active ingredient, the insulin may be present with an additional active ingredient such as a sulfonylurea. However, such active ingredients are generally administered separately in order to more closely control dosing and serum glucose levels.

Regardless of the active ingredient, there are several basic types of insulin formulations which can be used in connection with the present invention. All of the formulations include insulin, preferably with a pharmaceutically acceptable carrier suitable for intrapulmonary administration. In accordance with a first formulation, a low boiling point, highly volatile propellant is combined with the active ingredient and a pharmaceutically acceptable excipient The active ingredient may, for example, be provided as a suspension or dry powder in the propellant, or the active ingredient may be dissolved in solution within the propellant. Both of these formulations may be readily included within a container which has a valve as its only opening. Since the propellant is highly volatile, i.e., has a low boiling point, the contents of the container will be under pressure. Thus, when low boiling point propellants are used, the propellants are held within a pressurized canister of the device and maintained in a liquid state. When the valve is actuated, the propellant is released and forces the active ingredient from the canister along with the propellant. The propellant will "flash" upon exposure to the surrounding atmosphere, i.e., the propellant immediately evaporates. The flashing occurs so rapidly that it is essentially pure active ingredient which is actually delivered to the lungs of the patient The "flashing" phenomenon which occurs with the use of low boiling point propellants may greatly increase the practicality of the present invention as compared with nebulizers or formulations which do not use such propellants in that larger amounts of drug can be easily administered in a short period of time. Further, since the material being delivered to the lungs is essentially pure drug, it is easier to monitor and more closely control dosing which is a critical feature of the methodology of the present invention. Accordingly, when using such a delivery device it is preferable to use low boiling point propellants such as low boiling point chlorofluorocarbons or hydrocarbons, e.g., trichlorofluoromethane and dichlorodifluoromethane. As non-chlorofluorocarbon containing propellants are developed which are low boiling point propellants, their use in connection with the present invention will become apparent to those skilled in the art.

In accordance with a second formulation, the insulin is provided in a solution formulation. In this embodiment a dry powder is preferably dissolved in an aqueous solvent to create a solution which is moved through a porous membrane to create an aerosol for inhalation. Such solutions can be of the type which are made available commercially for injection and/or other solutions which are more acceptable for intrapulmonary delivery. An example of a suitable solution for generating aqueous aerosols from a nebulizer is the 0.9% saline solution disclosed in Laube, US patent 5,320,094.

A preferred form for inhaled administration of insulin is as a dry powder. Preferred insulin dry powders include those which are described in US patent 5,997,848 to Patton et al. Such insulin powders comprise free flowing particulates having a size selected to permit penetration into the alveoli of the lungs, generally being less than 10 µm in diameter, preferably less than 7.5 µm, most preferably less than 5 µm, and usually being in the range from 0.1 µm to 5 µm in diameter. Preferably, the insulin particle size is in the range of 0.5 to 3.5 µm. The aforementioned particle sizes generally apply to solid particles. It is also feasible to employ larger size particles which are aerodynamically light but which have a mean diameter much larger than 10 um, say 5 to 30 um. Such particles generally have a low tap density, less than 0.4 g/cm, and a mean diameter between 1 and three microns. Such particles are disclosed in US patent RE37,053 E, and in PCT published application WO 01/13891, both herein incorporated by reference. In any case, the insulin powder employed should be of a size that is adapted to penetrating to the deep lung where it can be absorbed through the alveoli.

Alternatively, amorphous insulins could be prepared by lyophilization (freeze-drying), vacuum drying, or evaporative drying of a suitable insulin solution under conditions to produce the amorphous structure. The amorphous insulin so produced can then be ground or milled to produce particles within the desired size range. Crystalline dry powder insulins may be formed by grinding or jet milling of bulk crystalline insulin. The preferred method for forming insulin powders comprising particulates in the desired size range is spray drying, where pure, bulk insulin (usually in a crystalline form) is first dissolved in a physiologically acceptable aqueous buffer, typically a citrate buffer having a pH in the range from about 2 to 9. The insulin is dissolved at a concentration from 0.01% by weight to 1 % by weight, usually from 0.1% to 0.2%. The solutions may then be spray dried in conventional spray drying equipment from commercial suppliers, such as Buchi, Niro, and the like, resulting in a substantially amorphous particulate product.

The dry insulin powders may consist essentially of insulin particles within the requisite size range and be substantially free from any other biologically active components, pharmaceutical carriers, and the like. Such "neat" formulations may include minor components, such as preservatives, present in low amounts, typically below 10% by weight and usually below 5% by weight. Using such neat formulations, the number of inhalations required for even high dosages can be substantially reduced, often to only a single breath.

The insulin powders useful in the present invention may optionally be combined with pharmaceutical carriers or excipients which are suitable for respiratory and pulmonary administration. Such carriers may serve simply as bulking agents when it is desired to reduce the insulin concentration in the powder which is being delivered to a patient, but may also serve to enhance the stability of the insulin compositions and to improve the dispersibility of the powder within a powder dispersion device in order to provide more efficient and reproducible delivery of the insulin and to improve handling characteristics of the insulin such as flowability and consistency to facilitate manufacturing and powder filling.

Suitable carrier materials may be in the form of an amorphous powder, a crystalline powder, or a combination of amorphous and crystalline powders. Suitable materials include carbohydrates, e.g. monosaccharides such as fructose, galactose, glucose, D-mannose, sorbose, and the like; disaccharides, such as lactose, trehalose, cellobiose, and the like; cyclodextrins, such as 2-hydroxypropyl-β-cyclodextrin; and polysaccharides, such as raffinose, maltodextrins, dextrans, and the like; (b) amino acids, such as glycine, arginine, aspartic acid, glutamic acid, cysteine, lysine, and the like; (c) organic salts prepared from organic acids and bases, such as sodium citrate, sodium ascorbate, magnesium gluconate, sodium gluconate, tromethamine hydrochloride, and the like; (d) peptides and proteins, such as aspartame, human serum albumin, gelatin, and the like; (e) alditols, such as mannitol, xylitol, and the like. A preferred group of carriers includes lactose, trehalose, raffinose, maltodextrins, glycine, sodium citrate, tromethamine hydrochloride, human serum albumin, and mannitol.

Such carrier materials may be combined with the insulin prior to spray drying, i.e., by adding the carrier material to the buffer solution which is prepared for spray drying. In that way, the carrier material will be formed simultaneously with and as part of the insulin particles. Typically, when the carrier is formed by spray drying together with the insulin, the insulin will be present in each individual particle at a weight percent in the range from 5% to 95%, preferably from 20% to 80%. The remainder of the particle will primarily be carrier material (typically being from 5% to 95%, usually being from 20% to 80% by weight), but will also include buffer(s) and may include other components as described above. The presence of carrier material in the particles which are delivered to the alveolar region of the lung (i.e., those in the requisite size range below 10 µm) has been found not to significantly interfere with systemic absorption of insulin.

Alternatively, the carriers may be separately prepared in a dry powder form and combined with the dry powder insulin by blending. The separately prepared powder carriers will usually be crystalline (to avoid water absorption), but might in some cases be amorphous or mixtures of crystalline and amorphous forms. The size of the carrier particles may be selected to improve the flowability of the insulin powder, typically being in the range from 25 µm to 100 µm. Carrier particles in this size range will generally not penetrate into the alveolar region of the lung and will often separate from the insulin in the delivery device prior to inhalation. Thus, the particles which penetrate into the alveolar region of the lung will consist essentially of insulin and buffer. A preferred carrier material is crystalline mannitol having a size in the above-stated range.

The dry insulin powders useful in the present inventions may also be combined with other active components. For example, it may be desirable to combine small amounts of amylin or active amylin analogues in the insulin powders to improve the treatment of diabetes. Amylin-is a hormone which is secreted with insulin from the pancreatic β-cells in normal (non-diabetic) individuals. It is believed that amylin modulates insulin activity in vivo, and it has been proposed that simultaneous administration of amylin with insulin could improve blood glucose control. Combining dry powder amylin with insulin in the compositions of the present invention will provide a particularly convenient product for achieving such simultaneous administration. Amylin may be combined with insulin at from 0.1% by weight to 10% by weight (based on the total weight of insulin in a dose), preferably from 0.5% by weight to 2.5% by weight. Amylin is available from commercial suppliers, such as Amylin Corporation, San Diego, Calif., and can be readily formulated in the compositions of the present invention. For example, amylin may be dissolved in aqueous or other suitable solutions together with the insulin, and optionally carriers, and the solution spray dried to produce the powder product.

The dry powder insulin compositions of the present invention are preferably aerosolized by dispersion in a flowing air or other physiologically acceptable gas stream in a conventional manner. One system suitable for such dispersion is described in copending application Ser. No. 07/910,048, which-has been published as WO 93/00951, the full disclosures of which are incorporated herein by reference. The full operation of such a device is disclosed therein.

A preferred dry powder formulation, particularly for use with the aforementioned inhaler, and which was used in the study described below, is disclosed in WO 98/16205 (the full text of which is herein incorporated by reference) as example 3. It consists of a dry powder made by spray drying a formulation containing 7.50 mg insulin, 1.27 mg mannitol, 3.38 mg sodium citrate, 0.026 mg sodium hydroxide, and 0.32 mg glycine per milliliter of deionized water, for a total solids concentration of 12.5 mg/mL at pH 7.3, the formulation being spray dried to produce a dry powder having an average particle size less than 5 µm. The powder is delivered to the deep lung via an inhaler. The preferred mode of treatment with dry powder insulin is as described in the aforementioned US patent 5,997,848.

### C. Tests

The tests used herein to assess the state of a patient, and whether or not a regimen of inhaled insulin is appropriate, are the well known, art-recognized measurement of fasting and/or random serum, plasma, or whole blood glucose measurements. Using such methods, glucose concentrations and plasma or serum insulin concentrations (the latter usually measured by radioimmunoassay or related techniques) are sampled before and after the administration of a known amount of glucose orally or intravenously. There are known normal patterns of glucose/insulin response to glucose challenge. Normal fasting glucose levels normally fall within the range of 80-126 milligrams per deciliter, mg/dl. Glucose levels in excess of 126 mg/dl may be sufficient for initiating a patient on inhaled insulin or switching a patient from a therapeutic regimen of subcutaneous insulin to inhaled insulin. Laboratory methods for measuring glucose and insulin levels are widely commercially available.

The amount of insulin to be administered via inhalation and the appropriate regimen will generally be determined by the attending physician. In general, when administering insulin in the form of an aerosol, as recombinantly produced human insulin plus excipients, a patient will be administered an amount of inhaled insulin in the range of 0.5 to 50 mg per day, usually 0.5 to 25 mg per day, usually in 1 to 4 individual dry powder doses. The dosing event itself will usually include administering the required dose in one or more, usually 1 to 4, inhalations from a suitable inhaler or nebulizer. Regardless of the form in which the inhaled insulin is delivered, i.e., regardless of whether the insulin is delivered as a dry powder, as an aqueous aerosol produced by a nebulizer, or as a suspension in a propellant delivered from a metered dose inhaler, a patient will be delivered an amount of insulin equivalent to between 1.5 and 150 units delivered systemically (1 mg of inhaled insulin being generally equivalent to about 3 Units (U) of systemically delivered recombinant human insulin). Insulin analogs which are superactive can be administered in substantially smaller amounts while obtaining substantially the same effect with respect to reducing serum glucose levels.

The effectiveness of inhaled insulin over subcutaneous insulin in reducing weight gain and improving fasting plasma glucose is exemplified by the following clinical study. The study was entitled EFFICACY AND SAFETY OF INHALED COMPARED WITH SUBCUTANEOUS HUMAN INSULIN THERAPY IN SUBJECTS WITH TYPE 2 DIABETES MELLITUS: A SIX-MONTH, OUTPATIENT, PARALLEL COMPARATIVE TRIAL.

The main objectives of the study were: In subjects with type 2 diabetes mellitus: 1. To determine whether glycemic control can be achieved at least as effectively with an inhaled insulin (INH) regimen as with a conventional subcutaneous (SC) insulin regimen. 2. To assess the toleration and safety of inhaled insulin, and its effects on measures of pulmonary function after 6 months' exposure. The study was designed as an open-label, randomized, 6-month (24-week), parallel outpatient study with a 4-week lead-in period in subjects with type 2 diabetes mellitus. After screening, subjects maintained a control SC insulin regimen consisting of twice daily (BID) administration of mixed regular insulin and NPH insulin. After the lead-in period, subjects were randomized to receive either the SC injected insulin regimen or a dry powder inhaled insulin regimen (pre-meal inhaled insulin (TID) plus a single bedtime Ultralente® injection) for the next 24 weeks. The inhaler employed was a dry powder inhaler obtained from Inhale Therapeutic Systems Inc., San Carlos, CA.

Male and female subjects aged 35-80 years inclusive with type 2 (not insulin-dependent) diabetes mellitus for >1 year, currently on a stable SC insulin administration schedule (at least BID for the 2 months prior to screening) were included in the study. Subjects were required to have screening and pre-randomization glycosylated hemoglobin (HbA1c) between 6% and 11%, inclusive. There was no requirement for specific fasting plasma glucose levels or weight, although entry with a Body Mass Index (BMI) greater than 35 required consultation with investigative staff. The following table shows the subject disposition.

| Evaluation Groups: | Inhaled | |
|---|---|---|
| | Insulin | SC Insulin |
| Randomized | 149 | 150 |
| Treated | 149 | 149 |
| Completed | 132 | 140 |
| Discontinued | 17 | 9 |
| Evaluated for Efficacy: Full Analysis Set (ITT) | 146 | 149 |
| Per Protocol Set (Evaluable) | 143 | 145 |
| Assessed for Safety: Adverse Events | 149 | 149 |
| Laboratory Tests | 135 | 142 |
| Pulmonary Function Tests | 145 | 145 |

The primary efficacy endpoint was the change in HbA1c from baseline to week 24 of treatment. HbA1c was collected at weeks -4, -1, 0, 6, 12, and 24. Secondary efficacy endpoints included change in fasting plasma glucose and body weight (week -4 and every 4 weeks thereafter) as well as percentage of subjects achieving acceptable glycemic control (HbA1c <8% or <7% at week 24), 2-hour post-prandial glucose and insulin increments following a standardized meal (baseline, week 24), fasting lipids (weeks 0 and 24), and home glucose monitoring. The incidence and severity of hypoglycemic events were monitored. A survey of treatment satisfaction and preference was also administered (weeks -4, -1, 6, 12, and 24).

Safety evaluations included: a full physical examination at screening and brief physical examination (including throat, chest, blood pressure, and heart rate) at week 0 and weeks 4, 8, 16, and 24; 12-lead ECG (screening and week 24); chest X-ray (screening, week 24); clinical laboratory safety tests (screening and week 24); insulin antibodies (weeks 0 and 24); and a pregnancy test for women of childbearing potential (screening). Comprehensive pulmonary function testing (spirometry, lung volumes, diffusion capacity, and oxygen saturation) was performed at baseline (week -3) and at week 24 (spirometry also at week 12). Observed and volunteered adverse events were recorded. At selected sites, a high resolution computerized tomography scan of the thorax was performed on a subset of subjects at baseline and week 24.

The following statistical methods: The primary efficacy endpoint was the week 24 change from baseline in HbA1c, which was analyzed for both the Full Analysis set (ITT), and the Per Protocol set (Evaluable: the primary analysis population). An analysis of covariance (ANCOVA) model with terms for baseline HbA1c, center, and treatment was fitted to the week 24 change from baseline HbA1c values. If the week 24 HbA1c was not available, the last evaluable post-baseline HbA1c value was carried forward (LOCF). Non-inferiority of inhaled insulin to subcutaneous insulin was concluded if the upper limit of the 95% confidence interval was less than 0.5%, the protocol-specified non-inferiority bound.

Treatment effects for continuous secondary efficacy parameters were estimated using an ANCOVA model similar to the primary model for HbA1c. The percent of subjects reaching pre-defined glycemic control goals (HbA1c <8% and <7%) at week 24 was analyzed using logistic regression. The hypoglycemic event risk ratio was estimated using the survival analysis counting process approach for recurrent events, where the analysis model included only a term for treatment.

Efficacy Results: Briefly, the two regimens showed similar general glycemic control with respect to HbA1c levels despite slightly lower over-all hypoglycemic risk (as evidenced by the 95% Confidence Interval (95%CI) for the adjusted Risk Ratio (RR) between the groups not crossing 1). However, the end-of-study fasting plasma glucose was 16 mg/dl less for the INH regimen as that seen with the SC regimen (as evidenced by the 95% Confidence Interval (95%CI) for the adjusted difference not crossing 0). Further, while the INH group gained on average 0.1 kg, the SC group gained 1.4 kg and the adjusted difference between the group, -1.3 kg, was significant (as evidenced by the 95% Cl for the adjusted difference not crossing 0).

| | | | | | | | | |
|---|---|---|---|---|---|---|---|---|
| Except where indicated, results in the following tables are shown for the Evaluable analysis set. | | | | | | | | |

| Mean Baseline (BL), Value at Week 24 (LOCF), and Mean Change from BL at Week 24 (LOCF) | | | | | | | | |
|---|---|---|---|---|---|---|---|---|
| | Inhaled Insulin (INH) | | | SC Insulin | | | Adj. INH-SC | |
| | BL | Wk 24 | Change | BL | Wk 24 | Change | Difference^{a} | 95% Cl^{a} |
| Primary Efficacy | | | | | | | | |
| HbA1c (%) | 8.1 | 7.4 | -0.7 | 8.2 | 7.6 | -0.6 | -0.07 | -0.32, 0.17 |
| HbA1c(%); (ITT set) | 8.1 | 7.4 | -0.7 | 8.2 | 7.6 | -0.6 | -0.07 | -0.31,0.17 |

| Secondary Efficacy | | | | | | | | |
|---|---|---|---|---|---|---|---|---|
| Fasting Glucose (mg/dL) | 152 | 132 | -20 | 158 | 149 | -9 | -15.88 | -26.61, -5.15 |
| Glucose Increment^{b} | 89 | 90 | 1 | 94 | 83 | -11 | 6.58 | -8.79, 21.94 |
| Body Weight (kg) | 90.5 | 90.5 | 0.1 | 89.2 | 90.6 | 1.4 | -1.29 | -1.98, -0.59 |
| Hypoglycemia | | # Events | Rate | | # Events | Rate | INH/SC Risk Ratio | |
| Total Events^{c} | | 1104 | 1.4 | | 1278 | 1.6 | 0.89 | 0.82, 0.97 |
| Severe Events^{d} | | 4 | 0.5 | | 1 | 0.1 | 4.07 | 0.46, 36.43 |

| | | | | | | | | |
|---|---|---|---|---|---|---|---|---|
| ^{a}Difference in adjusted mean change from baseline, and 95% Cl based on primary model. | | | | | | | | |
| ^{b}Change in concentration (mg/dL) between -30 min and 2 hours post-prandial, measured in standardized meal study. | | | | | | | | |
| ^{c}Crude event rate was calculated as number of events/total subject-months exposure. | | | | | | | | |
| ^{d}Crude event rate was calculated as number of events/100 subject-months exposure. | | | | | | | | |

| Baseline and End-of-Study HbA1c; Number (%) of Subjects | | | | |
|---|---|---|---|---|
| HbA1c: | <7% | 7% to <8% | 8% to <9% | ≥9% |
| Inhaled insulin (n=143) | | | | |
| Baseline | 25 (17.5) | 45 (31.5) | 47 (32.9) | 26 (18.2) |
| End of Study | 67 (46.9) | 42 (29.4) | 15 (10.5) | 19 (13.3) |

| SC insulin (n=145) | | | | |
|---|---|---|---|---|
| Baseline | 17 (11.7) | 51 (35.2) | 45 (31.0) | 32 (22.1) |
| End of Study | 46 (31.7) | 54 (37.2) | 33 (22.8) | 12 (8.3) |

| Safety Results are tabulated as follows: | | | | |
|---|---|---|---|---|
| Number of Subjects Evaluated (With Event) [Disc'd Due to Event] | | | | |
| | | Inhaled Insulin | | SC Insulin |
| All-causality adverse events^{a} | | 149 (141) [2] | | 149 (143) [2] |
| T/R adverse events, per investigator^{b} | | 149 (126) [2] | | 149 (118) [0] |
| All-causality serious adverse events^{a} | | 149 (13) [0] | | 149 (12) [2] |
| Laboratory test abnormalities (normal baseline) | | 135 (43) [0] | | 142 (56) [0] |
| Laboratory test abnormalities (abnormal baseline) | | 114 (17) [0] | | 128 (13) [0] |
| Key: T/R, - treatment-related; Disc'd - discontinued | | | | |

| | | | | |
|---|---|---|---|---|
| ^{a}All-causality adverse events reported during study drug treatment or within a 1-day lag period | | | | |
| ^{b}Treatment-related adverse events, regardless of treatment-emergence or timing relative to study drug | | | | |

Two deaths occurred in this study; neither were treatment-related. There were 25 serious adverse events (SAEs). One of these (SC insulin group) was treatment-related. Two of the serious adverse events in the SC insulin group (and none in the inhaled insulin group) resulted in permanent discontinuation. Almost all subjects in both treatment groups experienced an adverse event during the study (all-causality and treatment-related). The majority of adverse events reported were of mild or moderate severity. There were 23 (15%) subjects with 30 severe events in the inhaled insulin group and 11 (7%) subjects with 16 severe events in the subcutaneous insulin group.

The most commonly reported adverse event was treatment-related hypoglycemia. In addition to reported hypoglycemia, some of the other reported treatment-related adverse events (e.g., asthenia, tremor, dizziness, sweating, headache) may have been manifestations of mild or relative hypoglycemia. In general, the number of subjects with adverse events by body system and treatment relatedness was balanced between the inhaled insulin and subcutaneous insulin groups. There were a greater number of treatment-related respiratory adverse events in the inhaled insulin group than in the subcutaneous insulin group. These events were almost entirely increased cough, whose incidence and prevalence was greater in the inhaled insulin group. The incidence of cough decreased considerably over the study period.

The mean changes from baseline in the pulmonary function test data (FEV1, FVC, TLC, and DLco) were small and similar for the 2 groups.

There were no notable findings for blood pressures, heart rate, physical examination, chest X-ray or ECGs. In both the inhaled insulin and SC insulin groups, there was a 12.2 mg/dL mean change in triglycerides; mean changes in total, HDL, and LDL cholesterol were very small.

This study is simply an example of the results seen with INH versus SC regimens. While the body weight and fasting plasma glucose results in other studies have not always reached statistically significant levels, they have consistently shown similar trends. For example, another very similar study in Type 1 (insulin dependent) diabetics aged 12-65 showed 0.2 kg less weight gain for the INH group than the SC group (not significant), and fasting plasma glucose of 38 mg/dl lower in the INH group than the SC group (significant). Again, these results were in the context of similar over-all glycemic control (as judged by end-of-study HbA1c values) and a slightly smaller hypoglycemic event rate.

## Claims

1. A method of reducing the rate of weight gain in a diabetic patient who is using exogenous insulin to control blood sugars and who is taking said insulin by the subcutaneous and/or transdermal route of administration, comprising administering said insulin to said patient by the pulmonary route.

2. A method as defined in claim 1, wherein said pulmonary insulin is delivered in the form of aerosolized insulin wherein the aerosol is atomized from a solution.

3. A method as defined in claim 1, wherein said pulmonary insulin is administered as a dry powder.

4. A method as defined in any one of the preceding claims, wherein said patient has been using exogenous insulin by a subcutaneous route.

5. A method as defined in any one of claims 1 to 3, wherein said patient has been using exogenous insulin by a transdermal route.

6. A method as defined in any one of claims 1 to 3, wherein said patient is to be newly initiated on insulin.

7. A method as defined in any one of the preceding claims, wherein said effective amount of pulmonary insulin is delivered by a propellant as a solution or suspension of said insulin from a metered dose inhaler.

8. A method of lowering the fasting glucose level in a diabetic patient who is using exogenous insulin to control blood sugars and who is taking said insulin by the subcutaneous and/or transdermal route of administration, comprising administering said insulin to said patient by the pulmonary route.

9. A method as defined in claim 8, wherein said pulmonary insulin is delivered in the form of aerosolized insulin wherein the aerosol is atomized from a solution.

10. A method as defined in claim 8, wherein said pulmonary insulin is administered as a dry powder.

11. A method as defined in any one of claims 8 to 10, wherein said patient has been using exogenous insulin by a subcutaneous route.

12. A method as defined in any one of claims 8 to 10, wherein said patient has been using exogenous insulin by a transdermal route.

13. A method as defined in any one of claims 8 to 10, wherein said patient is to be newly initiated on insulin.

14. A method as defined in any one of claims 8 to 13, wherein said effective amount of pulmonary insulin is delivered by a propellant as a solution or suspension of said insulin from a metered dose inhaler.

15. The use of insulin for the manufacture of a medicament for administration by the pulmonary route for reducing the rate of weight gain, or for lowering the fasting glucose level, in a diabetic patient who needs exogenous insulin to control blood sugars.

16. The use of claim 15, wherein the medicament is in the form of aerosolized insulin wherein the aerosol is atomised from a solution.

17. The use of claim 15, wherein the medicament is adapted for the pulmonary administration of insulin as a dry powder.

18. The use of claim 15, wherein the medicament is a metered dose inhaler adapted for the pulmonary delivery of insulin by a propellant as a solution or suspension of said insulin.

19. The use of any one of claims 15 to 18, wherein the patient has been using exogenous insulin by a subcutaneous or transdermal route.

20. The use of any one of claims 15 to 18, wherein the patient is to be newly initiated on insulin.
